# EUROPÄISCHE PATENTSCHRIFT

(11) **EP 0 630 227 B1**
(45) Veröffentlichungstag und Bekanntmachung des Hinweises auf die Patenterteilung: **14.04.1999**
(21) Anmeldenummer: 94902591.0
(22) Anmeldetag: 05.01.1994
(51) Int. Cl.: A61K 7/16

(54) **ZAHNPASTA**
TOOTHPASTE
PATE DENTIFRICE

(30) Priorität: 14.01.1993 CH 104/93
(43) Veröffentlichungstag der Anmeldung: 28.12.1994
(73) Patentinhaber: HAWE NEOS DENTAL Dr. H. V. WEISSENFLUH AG, 6934 Bioggio (CH)
(72) Erfinder: BAFFELLI, Gianni, CH-6950 Tesserete (CH); VON WEISSENFLUH, Beat, A., CH-9625 Gentilino (CH)
(74) Vertreter: AMMANN PATENTANWAELTE AG BERN
(86) Internationale Anmeldenummer: CH9400003
(87) Internationale Veröffentlichungsnummer: WO9415577

(56) Entgegenhaltungen:
- EP-A- 0 268 763
- EP-A- 0 528 756

## Beschreibung

Die vorliegende Erfindung betrifft eine Zahnpasta mit verbesserter und die Zahnhartsubstanz schonende-Reinigungswirkung.

Zahnpasten sind ein alltägliches Verbrauchsprodukt und als solches in grosser Anzahl bekannt. Im allgemeinen enthalten sie einen reinigenden Putzkörper, der in einer wässrigen Phase dispergiert wird, und diverse Zusatzstoffe wie Pigmente, Tenside, Verdicker, Konservierungsmittel, karieshemmende Fluorverbindungen, Aroma- und Geschmacksstoffe einschliesslich Süssstoffe und Salze sowie Farbstoffe. Diese Zusatzstoffe sollen eine Mundhygiene bewirken und die Akzeptanz der Zahnpasta, besonders bei Kindern, sicherstellen.

Die Reinigung der Zähne soll durch den Putzkörper bewirkt werden, der einerseits Verunreinigungen beim Reiben mit der Zahnbürste mechanisch entfernen soll, andererseits aber die Zahnhartsubstanz, welche gegen Verkratzen empfindlich ist, nicht zerkratzen darf. Man hat dieses Problem bisher dadurch zu lösen versucht, dass man in die Zahnpasta zwar harte Mineralien einarbeitete, diese aber in sehr feinverteilter Form einsetzte, um die Bildung von Kratzern in der Zahnhartsubstanz zu vermeiden.

Als Putzkörper sind in der Literatur beispielsweise und insbesondere die folgenden, feinteiligen Stoffe als bisher verwendet beschrieben, wobei die folgende Liste keinen Anspruch auf Vollständigkeit erhebt und nur zeigen will, dass das Problem der richtigen Wahl des Putzkörpers in Zahnpasten keinesfalls als gelöst gelten kann: Calciumcarbonat, gefällter Kalk, Schlämmkreide, Bimsstein, Calcium- und Magnesiumphosphat, Magnesiumcarbonat, Bariumcarbonat, Glaspulver, Zeolithe, Korallenkalk, Talk, Kaolin, Knochensubstanz, Kieselgur, Aluminiumoxid, Siliciumdioxid, pulverförmige synthetische Stoffe etc. in allen möglichen Abwandlungen usw. Eine Übersicht wird in der BE-A-406'912 gegeben, die übrigens vorschlägt, als Putzkörper Substanzen mit einer Härte zu verwenden, die nicht grösser als die Härte des Zahnschmelzes ist, insbesondere mit einer Mohs-Härte zwischen 2 und 3. Als Beispiele werden Glimmer, Asbest und andere Stoffe genannt, die in Pulverform keine scharfen Kanten, sondern ebene Flächen aufweisen.

Auch dieser Vorschlag konnte bisher nicht befriedigen, da relativ weiche Putzkörper nicht die erforderliche Reinigungswirkung im Verlaufe des Zähneputzens entfalten, welches mindestens etwa 5 Minuten dauern soll, in der Regel aber nur während 1 bis 2 Minuten pro Tag intensiv durchgeführt wird.

Aufgabe der Erfindung war es, eine neuartige Zahnpasta zu entwickeln, die nun eine ausreichende Reinigungswirkung bei verbesserter Schonung der Zahnhartsubstanzen auch während relativ kurzer Behandlungszeiten ausübt. Die erfindungsgemässe Zahnpasta ist im ersten unabhängigen Patentanspruch definiert, während besondere Ausführungsformen den Gegenstand von abhängigen Ansprüchen bilden.

Es wurde nun überraschenderweise gefunden, dass als Putzkörper in der Zahnpasta ein Gestein bestens geeignet ist, das in Form scharfkantiger Teilchen vorliegt, die bei mechanischer Beanspruchung unter den Bedingungen des Zähneputzens in kleinere, ebenfalls scharfkantige Teilchen zerfallen. Dabei üben die ursprünglich vorhandenen, relativ groben Teilchen mit einer Korngrössenordnung von ca. 1 bis 150 µm mit einem Hauptanteil bei etwa 20 µm eine sehr kurzdauernde, dafür aber intensive Reinigungswirkung aus und werden dann sofort in immer feinere Teilchen zerlegt, die dann eine erwünschte, schonende Polierwirkung bis hin zu einer Feinstpolierung entfalten. Auf diese Weise erhält man eine sogenannte "intelligent-dynamische" Reinigungs- und Polierwirkung, und nach Beendigung des Zähneputzens ist die Zahnoberfläche derart geglättet, dass die erneute Ablagerung von Rückständen auf den Zähnen erschwert ist. Ein gut geeigneter Putz- und Polierkörper mit diesen Eigenschaften ist Perlit, der weiter unten besprochen werden soll.

Zahnpasten für den täglichen Gebrauch, die solche Putzkörper enthalten, sind bisher nicht bekannt geworden. Die EP-A2-0'268'763 beschreibt ein Zahnpflegemittel zur vorbeugenden Zahnhygiene und bezeichnet dieses zuweilen als Zahnpasta; gemeint ist aber ein pastenförmiges, vom Zahnhygieniker ein- bis zweimal jährlich mit Hilfe rotierender Bürsten oder Näpfchen angewandtes Zahnpflegemittel. Das Mittel, welches in dieser Veröffentlichung beschrieben ist, enthält Perlit und daneben als wichtigsten Bestandteil speziell bereitete Fällungskieselsäure. Die Wirkung des Perlits wird weder beschrieben noch angedeutet, sie wurde nicht erkannt, und der Fachmann wird dazu gebracht anzunehmen, der Perlit könne durch beliebige andere Putzkörper ersetzt werden.

Die EP-A-0 528 756 (nachveröffentlicht, Veröffentlichungsdatum: 24,Februar 1993) betrifft ein Zahnpflegemittel der oben beschriebenen Art, d.h. eine Abrasivpaste. In dieser Patentanmeldung ist die Wirkung des Perlits im Rahmen der Zahnpflege und im Hinblick auf seine physikalisch-mechanischen Eigenschaften erkannt worden; die beschriebenen Mittel sind jedoch zwingend als wasserfrei angegeben und müssen einen Stabilisator in Form hydrophober pyrogener Kieselsäure enthalten. Es wird zudem angegeben, dass Perlit als Reinigungsmittel in Zahnpflegemitteln, die in wässriger Phase vorliegen, unbrauchbar ist, da er unbenetzt nicht wirksam ist und wegen seiner grossen Wasseraufnahme zu grosse Mengen an Tensiden benötigen würde.

Überraschenderweise wurde nun gefunden, dass Perlit und ähnliche Stoffe doch in wässriger Phase brauchbar sind, wenn eine Verbindung zwischen Perlitteilchen und Wasser hergestellt wird. Die Wirksamkeit des Perlits in wässriger Phase und die Stabilität der Zahnpasta als Ganzes wird erfindungsgemäss durch ein synergetisch wirkendes Gemisch aus besonderen Tensiden erreicht. Dieses Gemisch besteht aus extrem reinem Natriumlaurylsulfat, Natriumlaurylsarkosinat und einem Natriumtaurid. Die Wirkung des Perlits und die Stabilität und Viskosität der Zahnpasta werden durch eine ebenfalls reinigend wirkende pyrogene Kieselsäure, die hydrophil gemacht worden ist, als Binder, Strukturierungsmittel und thixotropisches Mittel noch unterstützt.

Perlit ist ein rhyolithisch-glasiges Gestein vulkanischen Ursprungs und ist grundsätzlich ein Natrium-Kalium-Aluminiumsilikat mit etwa 73 % SiO₂, 13 % Al₂O₃, 1 % Fe₂O₃, unter 1 % TiO₂, 1 %CaO, unter 0,5 % MgO, 3,3 % Na₂O, 4 % K₂O und 2 bis 4 % Wasser. Die Prozentangaben beziehen sich hier und auch in der ganzen vorliegenden Beschreibung auf das Gewicht, wenn nichts anderes angegeben ist. Durch Vermahlen erhaltene Teilchen dieses Gesteins haben die Eigenschaft, wegen ihres Wassergehaltes in der Hitze aufzusprengen. Dies scheint eine besondere Eigenschaft zu sein, die nur bei Perlit auftritt. Bei der vorliegenden Erfindung wird nur der gesprengte (expandierte) Perlit besprochen und verwendet.

Perlit besitzt wie die anderen Gesteine seiner Gruppe, z.B. Ignimbrit, Tuff, Trass, eine innere Zellstruktur, und die beim Mahlen und nach dem Klassieren verbleibenden Bruchstücke erinnern unter dem Mikroskop an zerbrochene Eierschalen, d.h. scharfkantige und meist gekrümmte schalenartige Gebilde. Beim weiteren Zerbrechen dieser Schalen entsteht ein verkleinertes Abbild dieser Schalen, bis hin zum feinen Pulver.

Bevorzugt wird in der erfindungsgemässen Zahnpasta eine durch Klassieren erhaltene Fraktion des Perlits eingesetzt, die Teilchen mit einer Grösse zwischen etwa 1 und 150 µm umfasst, wobei etwa 90 % im Bereich von 5 bis 90 µm liegen und die häufigste Grösse etwa 20 bis 25 µm beträgt. Die Wahl einer solchen Fraktion war überraschend, da die bisher bekannten Zahnpasten mit Reibkörpern ähnlicher chemischer Zusammensetzung, z.B. Bimsstein, solche mit einer Teilchengrösse von etwa 1 bis 10 µm mit einer grössten Häufigkeit von ca. 3 bis 4 µm aufweisen.

Die als Stabilisator und Thixotropiemittel wirkende pyrogene Kieselsäure ist auch als Rauchkieselsäure ("Aerosil") bekannt und lässt sich beispielsweise auf bekannte Weise durch thermische Zersetzung von Siliciumtetrachlorid in einer Knallgasflamme und in Gegenwart von Wasser erhalten. Man erhält amorphe, kugelförmige Teilchen mit einem Durchmesser von 20 bis 40 nm und einer spezifischen Oberfläche von 100 bis 4oo m²/g. Durch eine Reaktion von oberflächlichen SiOH-Gruppen mit geeigneten Reagenzien kann die pyrogene Kieselsäure auf bekannte Weise hydrophil gemacht werden. Die erfindungsgemässe Zahnpasta enthält diese hydrophile pyrogene Kieselsäure, die handelsüblich ist, im allgemeinen im Gewichtsverhältnis von 12:1 bis 1:3, angegeben als Verhältnis von Perlit zur pyrogenen Kieselsäure.

Die erfindungsgemässe Zahnpasta enthält, wie bereits oben kurz erwähnt wurde, weitere Zusätze, die die Anpassung an Eignung und Akzeptanz bewirken sollen und auch zusätzliche Effekte ergeben. So kann die Zahnpasta wie heute allgemein üblich Fluorverbindungen zur Kariesprophylaxe enthalten; die verwendbaren Fluorverbindungen sind dem Fachmann bekannt. Weiterhin übliche Zusätze sind Benetzungsmittel wie Glycerin, Sorbit und andere Zucker, z.B. Disaccharide und/oder Zuckeralkohole sowie Polyethylenglycole, die auch als Süssstoffe bzw. Strukturierungsmittel wirken können; echte, natürliche oder synthetische Süssstoffe wie Saccharin oder süssende Peptide usw.; Binde- und Verdickungsmittel, z.B. Celluloseester und -ether wie Carboxymethylcellulosen; Konservierungsmittel wie Hydroxybenzoate; Farbstoffe, Aromastoffe wie Pfefferminzöl, Pigmente und in Sonderfällen auch andere Reibkörper wie Calciumcarbonat. Alle diese Zusätze sind im allgemeinen üblich und dem Fachmann bekannt.

Eine bevorzugte erfindungsgemässe Zahnpasta ist gewichtsmässig wie folgt zusammengesetzt:

| | |
|---|---|
| wässrige Flüssigkeit aus Wasser sowie Glycerin, Sorbit, Disaccharidsirup usw. | 55 bis 85 % |
| Süssstoff | 0,1 bis 0,5 % |
| organischer Verdicker | 0 bis 3 % |
| Polyethylenglycol | 0 bis 6 % |
| Konservierungsmittel, Fluorverbindungen, Farb- und Aromastoffe, Pigmente | 0,5 bis 12 % |
| Tensidgemisch, wie oben definiert | 2 bis 5 % |
| Perlit, mittl. Teilchengrösse ca. 20 bis 25 µm | 1 bis 12 % |
| pyrogene Kieselsäure, hydrophil | 1 bis 3 %. |

Für die Langzeitverwendung einer Zahnpasta sind deren Abrasiveigenschaften von besonderer Bedeutung, denn es darf nicht vorkommen, dass durch eine regelmässige Benutzung der Zahnpasta die Zahnhartsubstanz beschädigt wird. Es wurden daher empfindliche Messverfahren eingeführt und Standardwerte der Abrasion ermittelt, die eine höchstzulässige Abtragung des Substrats bzw. der Zahnhartsubstanz, auf die die Zahnpasta einwirkt, definiefen.

Eine grobe Orientierung über die Abrasion erhält man durch Behandeln von Kupferplatten durch eine Zahnpasta unter definierten Bedingungen und anschliessende Bestimmung des Kupferverlustes. Diese Methode ist allerdings zu ungenau.

Heute hat sich die sogenannte RDA/REA-Methode eingeführt. Sie beruht auf einer Messung von Radioaktivität. Definiert radioaktiv gemachte extrahierte menschliche oder tierische Zähne werden mit der zu prüfenden Zahnpasta bearbeitet, und die Radioaktivität der in Suspension überführten Zahnpasta nach Bearbeitung wird gemessen. Je höher die Werte sind, desto grösser war der Abrieb. Der RDA-Wert bezieht sich auf das Zahnbein (Dentin), der REA-Wert auf die Zahnhartsubstanz (Email). Die Methode ist im Journal of Dental Research 55 (4), 563 (1976) angegeben.

Es wurden nun mit der erfindungsgemässen Zahnpasta Messungen angestellt, wobei eine bekannte, marktgängige Zahnpasta als Vergleich diente. Die Vergleichspasta, im folgenden Paste V genannt, hatte einen Putzkörper mit einer Teilchengrösse von 3 bis 4 µm (Hauptanteil). Der für Paste V gemessene RDA-Wert betrug 82, der mit der erfindungsgemässen Zahnpasta, welche 6 % Perlit enthielt, erhaltene Wert 67. Allerdings hatte die erfindungsgemässe Zahnpasta eine Reinigungskraft von 155, gemessen nach einer konventionellen Methode während 30 Sekunden, während die Paste V eine solche von 100 aufwies. Da man die Zahnhartsubstanzabtragung natürlich auf gleiche Reinigungskraft beziehen muss, besitzt also die erfindungsgemässe Zahnpasta einen relativen RDA-Wert von ca. 43 und die Paste V von 82. Der Abrieb, verursacht durch die erfindungsgemässe Zahnpasta, ist also nur etwa halb so gross wie derjenige der Vergleichspasta.

Eine weitere erfindungsgemässe Zahnpasta, welche lediglich 3 % Perlit enthielt und sonst wie die oben untersuchte Zahnpasta zusammengesetzt war, hatte einen RDA-Wert von etwa 20, während die Reinigungskraft nur um etwa 25 % zurückgegangen war. Mit dieser Zahnpasta ergeben sich also noch viel günstigere Abriebverhältnisse.

Die erfindungsgemässe Zahnpasta kann im Rahmen der folgenden Patentansprüche weiter verändert und vervollkommnet werden. So ist es zum Beipiel möglich, einen weiteren Reibkörper einzuarbeiten; solche sekundären Reibkörper, die bereits bekannt sind, können der vorliegenden Beschreibung entnommen werden.

## Patentansprüche

1. Zahnpasta für die tägliche Zahnpflege mit verbesserter und die Zahnhartsubstanz schonender Reinigungswirkung, enthaltend eine wässrige Flüssigkeit und einen Reibkörper,
wobei
- die Zahnpasta weniger als 15 Gew.-% Fällungskieselsäure enthält oder frei von Fällungskieselsäure ist,
- Perlit ein Bestandteil des Reibkörpers und in einer Menge von 1 Gew.-% bis 12 Gew.-% der Zahnpasta in selbiger vorhanden ist und
- das Perlit in Form scharfkantiger Teilchen vorliegt, die bei mechanischer Beanspruchung unter den Bedingungen des Zähneputzens in kleinere, ebenfalls scharfkantige Teilchen zerfallen.

2. Zahnpasta nach Anspruch 1, dadurch gekennzeichnet, dass der genannte Reibkörper eine Perlitfraktion ist.

3. Zahnpasta nach Anspruch 2, dadurch gekennzeichnet, dass sie die Perlitfraktion als alleinigen Reibkörper enthält.

4. Zahnpasta nach Anspruch 1 oder 2, dadurch gekennzeichnet, dass sie weiterhin einen sekundären Reibkörper enthält.

5. Zahnpasta nach einem der Ansprüche 1 bis 3, dadurch gekennzeichnet, dass sie weiterhin als Binder und Viskositätsregler eine feinteilige pyrogene, hydrophil gemachte Kieselsäure enthält.

6. Zahnpasta nach einem der Ansprüche 2 bis 5, dadurch gekennzeichnet, dass sie als Perlitfraktion eine solche enthält, welche eine häufigste mittlere Teilchengösse von 20 bis 25 µm besitzt, wobei Anteile unter 1 µm und über 150 µm abwesend sind und die Fraktion zwischen 20 und 25 µm etwa 50 Gew.-% der Gesamtfraktion beträgt.

7. Zahnpasta nach einem der Ansprüche 1 bis 6, dadurch gekennzeichnet, dass sie 1 bis 6 Gew.-% an Perlit enthält.

8. Zahnpasta nach einem der Ansprüche 1 bis 6, dadurch gekennzeichnet, dass sie 1 bis 3 Gew.-% an Perlit enthält.

9. Zahnpasta nach einem der Ansprüche 5 bis 8, dadurch gekennzeichnet, dass das Gewichtsverhältnis von Perlit zur pyrogenen Kieselsäure etwa 12:1 bis 1:3 beträgt.

10. Zahnpasta nach einem der vorstehenden Ansprüche, dadurch gekennzeichnet, dass sie ein Tensidgemisch enthält, zusammengesetzt aus extrem reinem Natriumlaurylsulfat, Natriumlaurylsarcosinat und einem Natriumtaurid.

11. Zahnpasta nach einem der vorstehenden Ansprüche, dadurch gekennzeichnet, dass sie die folgende gewichtsmässige Zusammensetzung aufweist:
| | |
|---|---|
| wässrige Flüssigkeit aus Wasser sowie Glycerin, Sorbit usw. | 55 bis 85 % |
| künstlicher Süssstoff | 0,1 bis 0,5 % |
| organischer Verdicker | 0 bis 3 % |
| Polyethylenglycol | 0 bis 6 % |
| Konservierungsmittel, Fluorverbindungen, Farb- und Aromastoffe, Pigmente | 0,5 bis 12 % |
| Tensidgemisch gemäss Anspruch 10 | 2 bis 5 % |
| Perlit, mittl. Teilchengrösse ca. 20 bis 25 µm | 1 bis 12 % |
| pyrogene Kieselsäure, hydrophil | 1 bis 3 %. |

## Claims

1. Toothpaste for daily dental care, having improved cleaning power and being easy to the hard dental substance, containing an aqueous liquid and an abrasive body, the toothpaste containing less than 15 % by weight of precipitated silicic acid or being free from precipitated silicic acid, perlite being present as an abrasive body component in an amount of from 1 to 12 % by weight of the toothpaste, said perlite being in the form of sharp-edged particles which disintegrate by mechanical stress under the conditions of toothbrushing into smaller, also sharp-edged particles.

2. Toothpaste according to claim 1, characterised in that said abrasive body is a perlite fraction.

3. Toothpaste according to claim 2, characterised in that it contains the perlite fraction as the sole abrasive body.

4. Toothpaste according to claim 1 or 2, characterised in that it further contains a secondary abrasive body.

5. Toothpaste according to any one of claims 1 to 3, characterised in that it further contains a finely divided pyrogenic silicic acid which has been made hydrophilic, as a binder and a viscosity regulator.

6. Toothpaste according to any one of claims 2 to 5, characterised in that it contains as a perlite fraction such a fraction which has a most frequent average particle size of from 20 to 25 µm, particles having sizes below 1 µm and exceeding 150 µm being absent, and that the fraction between 20 and 25 µm amounts to about 50 % by weight of the total perlite fraction.

7. Toothpaste according to any one of claims 1 to 6, characterised in that it contains from 1 to 6 % by weight of perlite.

8. Toothpaste according to any one of claims 1 to 6, characterised in that it contains from 1 to 3 % by weight of perlite.

9. Toothpaste according to any one of claims 5 to 8, characterised in that the weight ratio of perlite to pyrogenic silicic acid is comprised between about 12:1 to 1:3.

10. Toothpaste according to any one of the preceding claims, characterised in that it contains a tenside mixture composed of extremely pure sodium lauryl sulfate, sodium lauryl sarcosinate and a sodium taurid.

11. Toothpaste according to any one of the preceding claims, characterised in that it has the following composition, given by weight:
| | |
|---|---|
| aqueous liquid of water, glycerol, sorbitol, etc. | 55 to 85% |
| artificial sweetening agent | 0.1 to 0.5% |
| organic thickening agent | 0 to 3% |
| polyethylene glycol | 0 to 6% |
| preservative, fluorine compounds, colouring and flavouring agents, pigments | 0.5 to 12% |
| tenside mixture according to claim 10 | 2 to 5% |
| perlite, av. particle size about 20 to 25 µm | 1 to 12% |
| pyrogenic silicic acid | 1 to 3%. |

## Revendications

1. Pâte dentifrice pour les soins dentaires quotidiens, ayant un effet nettoyant amélioré et ménageant l'émail dentaire, contenant un liquide aqueux et un matériau abrasif, la pâte dentifrice contenant moins de 15 % en poids d'acide silicique précipité ou n'en contenant pas du tout, le matériau abrasif contenant de la perlite qui est présente dans la pâte dentifrice à raison de 1 à 12 % en poids, cette perlite étant sous forme de particules à bords vifs se désintégrant, par sollicitation mécanique lors du brossage des dents, en particules plus petites ayant également des bords vifs.

2. Pâte dentifrice selon la revendication 1, caractérisée en ce que le dit matériau abrasif est une fraction de perlite.

3. Pâte dentifrice selon la revendication 2, caractérisée en ce qu'elle contient la fraction de perlite comme seul matériau abrasif.

4. Pâte dentifrice selon la revendication 1 ou 2, caractérisée en ce qu'elle contient en plus un matériau abrasif secondaire.

5. Pâte dentifrice selon l'une des revendications 1 à 3, caractérisée en ce qu'elle contient en plus un acide silicique pyrogéné fin et rendu hydrophile, à titre de liant et de régulateur de viscosité.

6. Pâte dentifrice selon l'une des revendications 2 à 5, caractérisée en ce qu'elle contient comme fraction de perlite une fraction ayant une grosseur moyenne de particules la plus nombreuse comprise entre 20 et 25 µm, cette fraction étant exempte de particules de moins de 1 µm et de plus de 150 µm, la fraction de particules entre 20 et 25 µm constituant environ 50% de la fraction totale.

7. Pâte dentifrice selon l'une des revendications 1 à 6, caractérisée en ce qu'elle contient de 1 à 6 % en poids de perlite.

8. Pâte dentifrice selon l'une des revendications 1 à 6, caractérisée en ce qu'elle contient de 1 à 3 % en poids de perlite.

9. Pâte dentifrice selon l'une des revendications 5 à 8, caractérisée en ce que le rapport pondéral entre la perlite et l'acide silicique pyrogéné est compris entre environ 12:1 et 1:3.

10. Pâte dentifrice selon l'une des revendications précédentes, caractérisée en ce qu'elle contient un mélange de surfactifs composé de laurylsulfate de sodium extrêmement pur, de laurylsarcosinate de sodium et d'un tauride de sodium.

11. Pâte dentifrice selon l'une des revendications précédentes, caractérisée en ce qu'elle présente la composition pondérale suivante:
| | |
|---|---|
| liquide aqueux comprenant de l'eau, de la glycérine, du sorbitol, etc. | 55 à 85 % |
| édulcorant artificiel | 0,1 à 0,5 % |
| épaississant organique | 0 à 3 % |
| polyéthylène glycol | 0 à 6 % |
| agent de conservation, composés fluorés, colorants, arômes, pigments | 0,5 à 12 % |
| mélange de surfactifs selon la revendivation 10 | 2 à 5 % |
| perlite, grosseur moyenne des particules environ 20 à 25 µm | 1 à 12 % |
| acide silicique pyrogéné, hydrophile | 1 à 3 %. |
